(19) Europäisches Patentamt
European Patent Office
Office européen des brevets

(11) Numéro de publication: **0 065 908**
**B1**

# (12) FASCICULE DE BREVET EUROPEEN

(45) Date de publication du fascicule du brevet: **05.12.84**

(51) Int. Cl.³: **C 07 D 401/06**, C 07 D 211/18, A 61 K 31/445

(21) Numéro de dépôt: **82400872.6**

(22) Date de dépôt: **11.05.82**

(54) **((Alkyl- et alcényl-3 pipéridyl-4)-2 éthyl)-3 indoles et leur utilisation comme médicaments.**

(30) Priorité: **22.05.81 FR 8110219**

(43) Date de publication de la demande:
**01.12.82 Bulletin 82/48**

(45) Mention de la délivrance du brevet:
**05.12.84 Bulletin 84/49**

(84) Etats contractants désignés:
**BE CH DE FR GB IT LI LU NL SE**

(56) Documents cités:
**EP - A - 0 007 258**
**FR - M - 6 291**
**GB - A - 925 429**
**GB - A - 1 023 781**

(73) Titulaire: **PHARMUKA LABORATOIRES, 35 Quai du Moulin de Cage, F-92231 Gennevilliers (FR)**

(72) Inventeur: **Le Fur, Gérard Roger, 35, rue du Progrès, F-92350 Plessis-Robinson (FR)**
Inventeur: **Renault, Christian Louis Albert, 10 Allée Réaumur, F-95150 Taverny (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 065 908 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

Le brevet français N° 75.38051 (N° 2334358) déposé le 12 décembre 1975 décrit des dérivés de l'indole, actifs comme inhibiteurs spécifiques de la recapture de la sérotonine par les neurones et pouvant de ce fait trouver des applications comme médicaments psychotropes, plus particulièrement antidépresseurs. Comme tels on peut mentionner spécialement le [(pipéridyl-4)-2 éthyl]-3 indole de formule:

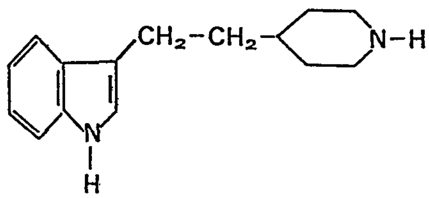

(I)

ou indalpine [G. Le Fur et coll., «Life Sciences», *23*, 1959 (1978)].

La présente invention a pour objet, à titre de produits nouveaux, les composés de formule générale:

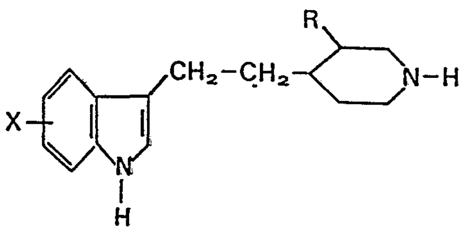

(II)

dans laquelle X représente un atome d'hydrogène ou d'halogène, par exemple le fluor ou le chlore, et R représente un groupe alkyle ou alcényle possédant 1 à 3 atomes de carbone.

Il a en effet été trouvé, conformément à la présente invention faite dans les services de la titulaire, que les composés de formule II possèdent non seulement, comme l'indalpine, la propriété d'inhiber la recapture de la sérotonine mais aussi la propriété de provoquer la libération de la sérotonine contenue soit dans les neurones, soit dans les plaquettes sanguines. Cette double fonction se manifeste par une action plus intense et plus rapide sur la dépression en raison de la libération neuronale de sérotonine dans la fente synaptique, conjuguée à l'inhibition de la recapture. La libération dans le plasma sanguin de la sérotonine plaquettaire améliore les états migraineux; enfin, la déplétion en sérotonine des plaquettes prévient la formation de thrombi artériels.

La molécule des composés de formule II comporte deux atomes de carbone asymétriques; il existe donc, pour une signification donnée de X et R, deux diastéréo-isomères, dénommés respectivement composé cis et composé trans selon que le groupe R et le groupe fixé en position 4 sur le cycle pipéridyle sont en position cis ou en position trans l'un par rapport à l'autre. Ces deux diastéréo-isomères peuvent être représentés schématiquement par les formules suivantes:

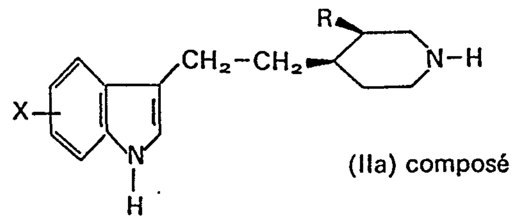

(IIa) composé cis

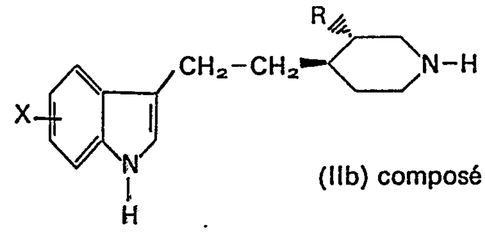

(IIb) composé trans

A chaque diastéréo-isomère correspondent un racémique et deux énantiomères, chaque énantiomère correspondant à une configuration absolue déterminée rectus (R) ou sinister (S) des carbones en position 3 et 4 du cycle pipéridyle.

Les divers isomères signalés ci-dessus et leurs mélanges font partie de l'invention.

Les composés de formule générale II peuvent être préparés par réaction d'un dérivé de l'indole de formule:

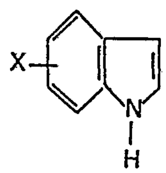

(III)

dans laquelle X a les mêmes significations que dans la formule II avec un dérivé métallique de formule R'Li, dans laquelle R' représente un groupe alkyle ou aryle ou un groupe dialkylamino, réaction du composé métallé ainsi obtenu (formule IV du schéma ci-dessous) avec un dérivé pipéridinique de formule:

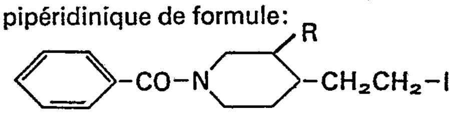

(V)

dans laquelle R a les mêmes significations que dans la formule II, et hydrolyse du composé ainsi obtenu (formule VI). L'ensemble des réactions peut être schématisé comme suit:

a)

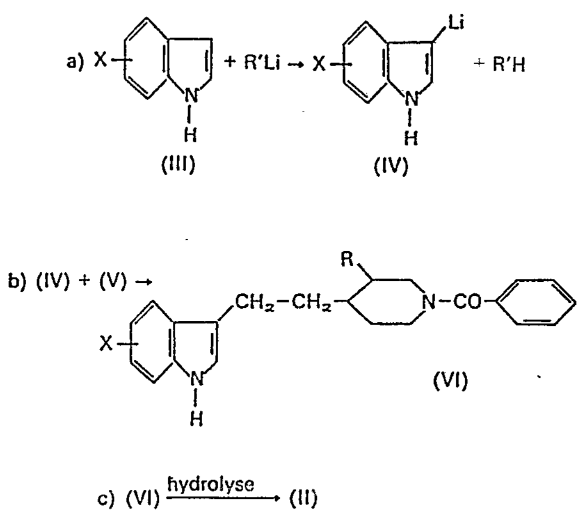

La réaction désignée ci-dessus a est effectuée au sein d'un solvant inerte: un éther tel que l'oxyde de diéthyle, le tétrahydrofuranne ou le diméthoxyéthane ou un hydrocarbure tels que le benzène, le toluène ou le xylène, ou un mélange de ces solvants, à une température comprise entre −10 et +20°C. Comme dérivé métallique R'Li, on utilise de préférence le phényllithium, le butyllithium ou le diisopropylamidure de lithium.

La réaction de condensation b est effectuée dans les mêmes solvants que la réaction a et à une température comprise entre 0°C et la température d'ébullition du solvant.

La réaction d'hydrolyse c, qui consiste à remplacer le groupe benzoyle par un atome d'hydrogène, est effectuée selon des méthodes connues en soi, par exemple au moyen de l'hydroxyde de sodium en milieu hydroorganique, tel qu'un mélange d'eau et d'éthylèneglycol ou un mélange d'eau et d'éther monométhylique de l'éthylèneglycol, à la température d'ébullition du milieu utilisé.

Les dérivés pipéridiniques de formule V peuvent être préparés selon le schéma réactionnel suivant:

d) 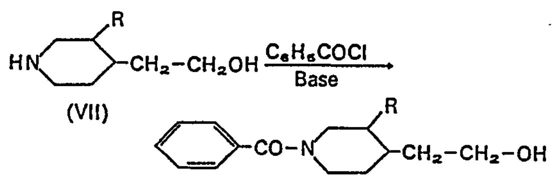

(VII)
(VIII)

e) 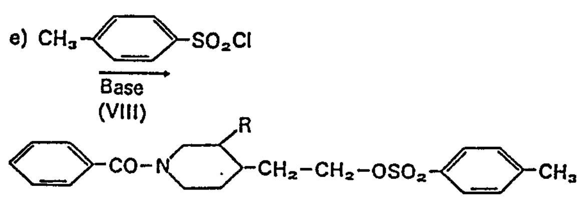

(VIII)
(IX)

f) (IX) $\xrightarrow{LiI}$ (V)

La réaction d du chlorure de benzoyle sur les alcools de formule VII est effectuée avantageusement en présence d'une base organique telle qu'une amine tertiaire, par exemple la triéthylamine, ou minérale, tels l'hydroxyde ou le carbonate de sodium ou de potassium en solution aqueuse. On opère au sein d'un solvant inerte tel que le chloroforme ou le trichloro-1,1,1 éthane à une température comprise entre 20°C et la température d'ébullition du solvant utilisé.

La réaction e du chlorure de paratoluènesulfonyle (chlorure de tosyle) avec les alcools de formule VIII est effectuée à une température comprise entre −20°C et la température ambiante, au sein d'un solvant inerte et en présence d'une base (de préférence une amine). Une méthode avantageuse consiste à opérer au sein de la pyridine qui joue à la fois le rôle de solvant et de base.

Les produits obtenus, de formule IX, sont ensuite traités (réaction f) par l'iodure de lithium au sein d'un solvant inerte, tel que l'acétone ou la méthyléthylcétone, à une température comprise entre 0 et 30°C.

Les produits de formule II peuvent également être préparés par réduction des dérivés cétoniques de formule générale:

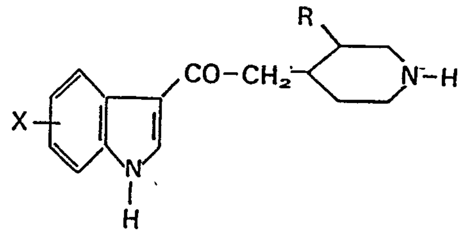
(X)

dans laquelle R et X ont les mêmes significations que dans la formule II.

Comme agent réducteur, on peut utiliser un hydrure métallique tel que le borohydrure de sodium, de potassium ou de lithium ou le triéthylborohydrure de lithium, ou encore l'hydrure d'aluminium et de lithium, au sein des solvants appropriés connus pour chacun de ces agents réducteurs, à des températures comprises entre 0°C et la température d'ébullition du solvant utilisé.

Les dérivés cétoniques de formule X peuvent être préparés selon le schéma réactionnel suivant:

g) 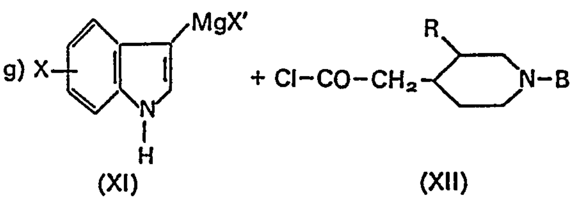
(XI) (XII)

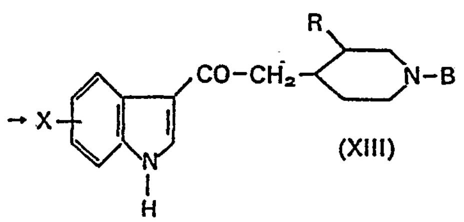
(XIII)

h) (XIII) $\xrightarrow{\text{acide}}$ (X)

Le réactif de Grignard de formule XI, dans laquelle X à la même signification que dans la formule II et X' représente un atome de chlore, de brome ou d'iode, est obtenu par action sur l'indole correspondant d'un halogénure d'alkylmagnésium tel que le chlorure, le bromure ou l'iodure de méthylmagnésium au sein d'un éther tel que l'éther diéthylique, généralement à la température d'ébullition du solvant. A la solution ainsi obtenue et refroidie à une température comprise entre −10 et +20°C, on ajoute (réaction g) le chlorure d'acide de formule XII dans laquelle B représente le groupe benzyloxycarbonyle et R a les mêmes significations que dans la formule II. On obtient ainsi les composés de formule XIII. La coupure du groupement benzyloxycarbonyle (réaction h) peut être effectuée par un acide tel que l'acide chlorhydrique au sein d'un solvant inerte tel que l'acide acétique ou l'éthanol à une température comprise

entre 20°C et la température d'ébullition du solvant utilisé.

Les chlorures d'acides de formule XII peuvent être préparés en 2 étapes à partir des acides pipéridyl-4 acétiques de formule XIV ci-dessous, dans laquelle R a les mêmes significations que dans la formule II suivant le schéma réactionnel suivant:

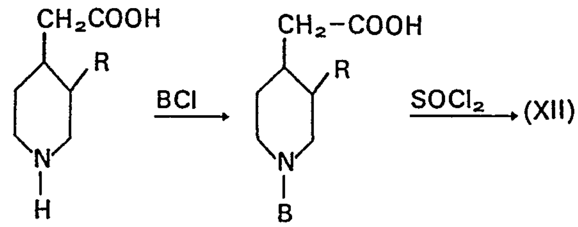


(XIV) (XV)

Les acides de formule XIV sont traités par le chloroformiate de benzyle (BCl) en solution dans un hydrocarbure en présence d'une base telle que l'hydroxyde de sodium en solution aqueuse à une température comprise entre 10 et 40°C. Les acides obtenus de formule XV sont transformés en chlorures d'acide de formule XII par action d'un agent de chloruration tel que le chlorure de thionyle au sein d'un solvant inerte tel que le chloroforme et à la température d'ébullition du solvant utilisé.

Les composés de formule II, dans laquelle R représente un groupe alkyle possédant 2 à 3 atomes de carbone, peuvent également être préparés par hydrogénation catalytique des produits correspondants de formule II dans laquelle R représente un groupe alcényle ou de leurs sels. Cette hydrogénation peut être réalisée à la pression atmosphérique, à une température comprise entre 20 et 50°C au sein d'un solvant inerte tel qu'un alcool (par exemple le méthanol ou l'éthanol) ou un acide (par exemple l'acide acétique) en présence d'un catalyseur tel que le palladium, le nickel, le rhodium, le ruthénium ou le platine.

Les composés de formule II, dans laquelle R représente le groupe vinyle et dont l'atome de carbone du cycle pipéridinique porteur du groupe vinyle a une configuration donnée rectus (R) ou sinister (S), peuvent être préparés par chauffage à une température supérieure à 50°C, au sein d'un solvant protique ou d'un mélange de solvants protiques, en présence ou en l'absence de formaldéhyde, des composés correspondants de formule II dans laquelle R représente le groupe vinyle et dont l'atome de carbone du cycle pipéridinique porteur du groupe vinyle a la configuration inverse, sinister (S) ou rectus (R), partiellement ou totalement salifiés, selon le procédé du brevet européen No 0005654. Le chauffage peut être réalisé en particulier dans un milieu aqueux acide de pH voisin de 6, à une température de 120 à 160°C.

Les mélanges réactionnels obtenus suivant les divers procédés décrits précédemment sont traités suivant des méthodes classiques, physiques (évaporation, extraction à l'aide d'un solvant, distillation, cristallisation, chromatographie, etc.) ou chimiques (formation de sel et régénération de la base, etc.), afin d'isoler les composés de formule II à l'état pur.

Les composés de formule II sous forme de bases libres peuvent éventuellement être transformés en sels d'addition avec un acide minéral ou organique par action d'un tel acide au sein d'un solvant approprié.

Les exemples suivants illustrent l'invention sans la limiter. Les données relatives aux spectres de résonance magnétique nucléaire (en abrégé RMN) figurant dans ces exemples concernent la résonance magnétique nucléaire des protons des composés à l'état de base en solution dans le deutérochloroforme (sauf mention contraire). Les déplacements chimiques δ sont mesurés en utilisant comme référence la tétraméthylsilane.

*Exemple 1:*

*[Vinyl-3(R) pipéridyl-4 (R)]-2 éthyl-3-indole*

A 10 g d'indole dans 150 ml de toluène sous azote, on introduit, à 0°C, 52 ml d'une solution 1,6M de butyllithium dans l'hexane. Ensuite, on ajoute 15 g de benzoyl-1 éthényl-3 (R) iodoéthyl-4 (S) pipéridine en solution dans 50 ml de toluène et porte le mélange réactionnel à 50°C pendant 20 h. On verse le milieu organique sur de l'acide chlorhydrique 2N, extrait l'insoluble avec du chlorure de méthylène, lave la solution organique à l'eau, la sèche sur du sulfate de magnésium et l'évapore à sec sous pression réduite. On obtient 21 g de produit que l'on chromatographie sur de la silice au moyen d'un mélange toluène/diéthylamine (80/20) comme éluant. On récupère 5,3 g de produit que l'on traite pendant 9 h par 50 ml d'une solution aqueuse d'hydroxyde de sodium 3N et 50 ml d'éther monométhylique de l'éthylèneglycol. On évapore les solvants sous pression réduite, partage le résidu entre l'eau et le chloroforme, lave la phase organique à l'eau, la sèche et l'évapore à sec. On récupère 2,7 g de produit que l'on recristallise dans de l'éthanol absolu, ce qui fournit 2 g de vinyl-3 (R) pipéridyl-4 (R)-2 éthyl-3 indole fondant à 168°C.

La benzoyl-1 vinyl-3 (R) iodoéthyl-4 (S) pipéridine est préparée en 3 étapes, de la façon suivante:

*Benzoyl-1 vinyl-3 (R) pipéridyl-4 (S) éthanol*

On porte 2 h au reflux un mélange de 20 g de vinyl-3 (R) pipéridyl-4 (S) éthanol, de 19,7 g de chlorure de benzoyle et de 108 g de carbonate de potassium dans 250 ml de chloroforme et 11 ml d'eau.

Après refroidissement, on filtre le mélange réactionnel et évapore le filtrat sous pression réduite. On chromatographie le résidu sur du gel de silice au moyen d'un mélange cyclohexane/acétate d'éthyle (7/3) comme éluant. On obtient ainsi 24 g de benzoyl-1 vinyl-3 (R) pipéridyl-4 (S) éthanol sous forme d'huile.

Spectre RMN du produit obtenu:

$C_6H_5$ δ: 7,4 ppm $-CH_2-OH$ δ: 3,6 ppm
$C\underline{H}_2 = CH-$ δ: 5 ppm
$CH_2 = \underline{C}H-$ δ: 6 ppm

Le vinyl-3 (R) pipéridyl-4 (S) éthanol peut être préparé selon R. Lukes, «Chem. Listy», *47*, 858 (1953).

*Benzoyl-1 vinyl-3 (R) [(méthyl-4 phényl) sulfonyloxyéthyl]-4 (S) pipéridine*

On agite pendant 2 h à −10°C 80 g de benzoyl-1 vinyl-3 (R) pipéridyl-4 (S) éthanol et 73 g de chlorure de p-toluènesulfonyle dans 1 l de pyridine. On verse le mélange réactionnel dans 3 l d'eau et extrait l'insoluble avec 2 l d'éther. On lave la phase organique avec 4 fois 1 l d'acide chlorhydrique 0,1 N et 5 fois 1 l d'eau, la sèche sur du sulfate de magnésium anhydre, la filtre et l'évapore à sec sous pression réduite. On obtient ainsi 66 g de benzoyl-1 vinyl-3 (R) [(méthyl-4 phényl) sulfonyloxyéthyl]-4 (S) pipéridine sous forme d'huile.

Spectre RMN du produit obtenu:

$C_6H_5$ δ: 7,4 ppm; $-C\underline{H}_2-O-SO_2-$ δ: 4 ppm

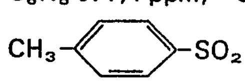 δ: 7,3 et 7,7 ppm

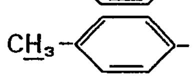 δ: 2,4 ppm

*Benzoyl-1 vinyl-3 (R) iodoéthyl-4 (S) pipéridine*

On agite pendant 24 h à la température ambiante 24,7 g de benzoyl-1 vinyl-3 (R) [(méthyl-4 phényl)sulfonyloxyéthyl]-4 (S) pipéridine et 16,4 g d'iodure de lithium dans 220 ml d'acétone. On filtre le mélange réactionnel, évapore le solvant sous pression réduite et redissout le résidu dans de l'acétate d'éthyle.

On lave la phase organique avec de l'eau, la sèche sur du sulfate de magnésium et l'évapore à sec sous pression réduite. On obtient ainsi 16 g de benzoyl-1 vinyl-3 (R) iodoéthyl-4 (S) pipéridine sous forme d'huile.

Spectre RMN du produit obtenu:

$C_6H_5$ δ: 7,4 ppm $-\underline{C}H_2-I$ δ: 3,2 ppm
$C\underline{H}_2 = CH-$ δ: 5 ppm
$CH_2 = C\underline{H}-$ δ: 6 ppm

*Exemple 2:*

*{[Ethyl-3 (R) pipéridyl-4 (R)]-2 éthyl}-3 indole*

A 17 g d'[éthyl-3 (R) pipéridyl-4 (S)]-2 (indolyl-3)-1 éthanone, dans 250 ml de tétrahydrofuranne anhydre, on ajoute, sous azote, à la température ambiante, 250 ml d'une solution molaire de triéthylborohydrure de lithium dans le tétrahydrofuranne. On porte le mélange réactionnel 18 h au reflux puis le refroidit à 0°C. On ajoute 300 ml d'eau et 400 ml d'éther éthylique, récupère la phase organique, la lave à l'eau, la sèche sur du sulfate de magnésium et l'évapore à sec sous pression réduite. On obtient 7 g de produit que l'on chromatographie sur du gel de silice au moyen d'un mélange méthanol/acétone/diéthylamine (5/5/0,5) comme éluant. Après cristallisation dans l'éther isopropylique, on obtient 3,7 g d'{[éthyl-3 (R) pipéridyl-4 (R)]-2 éthyl}-3 indole fondant à 150°C.

L'[éthyl-3 (R) pipéridyl-4 (S)]-2 (indolyl-3)-1 éthanone peut être préparé de la façon suivante:

A 14 ml d'une solution 3M d'iodure de méthylmagnésium dans l'éther, on ajoute, sous azote, une solution de 2,3 g d'indole dans 15 ml d'éther éthylique. On porte le mélange réactionnel pendant 1 h au reflux puis le refroidit à 0°C. A cette température, on ajoute 15 ml d'une solution 1,3M de chlorure d'acide benzyloxycarbonyl-1 éthyl-3 (R) pipéridine-4 (S) acétique dans l'éther éthylique. On agite pendant 24 h à la température ambiante, refroidit à 0°C et ajoute 60 ml d'éther éthylique et 60 ml d'acide chlorhydrique 2N. On récupère la phase organique, la lave à l'eau, la sèche et l'évapore à sec sous pression réduite. On obtient 7 g d'huile que l'on traite par 150 ml d'éthanol chlorhydrique 10N pendant 2 h au reflux. On évapore le solvant et chromatographie le résidu sur du gel de silice avec un mélange méthanol/acétone/diéthylamine (5/5/0,5) comme éluant. On obtient ainsi 2 g d'[éthyl-3 (R) pipéridyl-4 (S)]-2 (indolyl-3)-1 éthanone sous forme d'huile.

Spectre RMN du produit obtenu:

$C\underline{H}_3-CH_2$ δ: 0,9 ppm

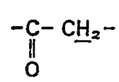 et 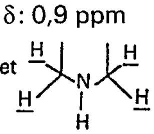 δ: 2,8 ppm

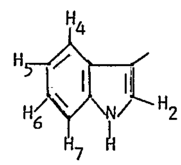

$H_2$ δ: 7,8 ppm; $H_4$ δ: 8,4 ppm; $H_5$, $H_6$, $H_7$ δ: 7,2 ppm.

Le chlorure de l'acide benzyloxycarbonyl-1 éthyl-3 (R) pipéridine-4 (S) acétique peut être obtenu de la manière suivante:

A 31 g de chlorhydrate de l'acide éthyl-3 (R) pipéridine-4 (S) acétique (cincholoïpon) dans 170 ml d'eau et 42 ml d'une solution aqueuse d'hydroxyde de sodium 10N, on ajoute à 10°C 62 ml d'une solution de chloroformiate de benzyle à 40% dans le toluène. On agite pendant 20 h à la température ambiante et lave la phase organique par 2 fois 250 ml d'éther éthylique. On acidifie la phase aqueuse avec de l'acide chlorhydrique concentrée et l'extrait avec 2 fois 250 ml de chloroforme. On sèche la phase organique sur du sulfate de magnésium anhydre et l'évapore à sec sous pression réduite. On obtient 45 g d'huile que l'on traite directement, au sein de 500 ml de chloroforme par 22 ml de chlorure de thionyle, pendant 2 h au reflux. On élimine le solvant sous pression réduite, on récupère 48 g de chlorure de l'acide éthyl-3 (R) pipéridine-4 (S) acétique que l'on utilise sous forme d'une solution 1,3M dans l'éther éthylique.

*Exemple 3:*

*{[Ethyl-3 (R) pipéridyl-4 (R)]éthyl}-3 indole*

On hydrogène pendant 3 h à la température ambiante et à pression atmosphérique 0,250 g de

{[vinyl-3 (R) pipéridyl-4 (R)]-éthyl}-3 indole dans 1 ml d'acide chlorhydrique normal et 9 ml d'éthanol en présence de 0,04 g d'oxyde de platine comme catalyseur. On filtre le catalyseur, évapore le filtrat à sec, reprend le résidu dans 100 ml d'eau, lave la phase aqueuse à l'éther, l'alcalinise avec une solution aqueuse d'hydroxyde de sodium et extrait l'insoluble avec de l'acétate d'éthyle. On sèche la phase organique sur du sulfate de magnésium et l'évapore à sec sous pression réduite. On obtient 0,180 g de produit fondant à 150°C.

*Exemple 4:*
*{[Vinyl-3 (S) pipéridyl-4 (R)]-2 éthyl}-3 indole*

On porte à 160°C pendant 48 h une solution de 1,36 g de [vinyl-3 (R) pipéridyl-4 (R)]-2 éthyl]-3 indole dans 210 ml d'eau et 5,3 ml d'acide chlorhydrique N. Après refroidissement, on alcalinise la solution au moyen de carbonate de potassium et l'extrait avec de l'acétate d'éthyle. On sèche la phase organique sur du sulfate de magnésium et l'évapore à sec sous pression réduite; on obtient 1,2 g de produit que l'on chromatographie sur de la silice au moyen d'un mélange toluène/diéthylamine (95/5) comme éluant. On obtient 0,45 g de produit que l'on recristallise dans de l'éthanol absolu, ce qui fournit 0,17 g de vinyl-3 (S) pipéridyl-4 (R)-2 éthyl-3 indole fondant à 168°C.

*Propriétés pharmacologiques*

On sait que la recapture de la sérotonine par les plaquettes sanguines constitue un bon modèle de la recapture de cette amine par les neurones [cf. J. Tuomisto, «J. Pharm., Pharmac.», *26*, 92 (1974)]. Appliquée à l'étude des médicaments, une méthode mettant en œuvre les plaquettes sanguines présente le grand intérêt de permettre l'utilisation de cellules humaines, ce qui lui confère un bon caractère prévisionnel.

La capacité des produits à inhiber la recapture de la sérotonine ou à provoquer sa libération a été mise en évidence sur des plaquettes sanguines humaines, selon J.L. David et coll., «Platelets Function and thrombosis, a review of methods», p. 335 (Plenum Press, London 1972).

1) *Inhibition de la recapture de la sérotonine*

Les résultats sont exprimés par une dose inhibitrice 50% ou $I_{50}$ qui représente la dose de produit, en micromoles par litre, diminuant de 50% la recapture de la sérotonine.

2) *Libération de la sérotonine*

L'action des produits sur la libération de la sérotonine est testée à la concentration de $5 \times 10^{-5}$ mol/l. Les résultats obtenus sont exprimés en pourcentage d'augmentation de la libération de la sérotonine par rapport aux résultats fournis par les témoins.

Les résultats obtenus avec les composés selon l'invention sont rassemblés dans le tableau. Dans ce tableau figurent, à titre comparatif, les résultats obtenus avec deux produits de référence (imipramine et p-chloroamphétamine) et avec l'indalpine.

*Tableau*

| Produit | Inhibition de la recapture de la sérotonine $I_{50}$ (µmol/l'') | Pourcentage d'augmentation de la libération de la sérotonine (concentration du produit: $5 \times 10^{-5}$ mol/l) |
|---|---|---|
| Exemple 1 | 0,008 | 84 |
| Exemple 2 | 0,06 | 75 |
| Exemple 4 | 0,035 | 91 |
| Indalpine | 0,035 | 22 |
| Imipramine | 0,4 | 13 |
| p-Chloro-amphétamine | 12 | 51 |

Il ressort du tableau que les produits de l'invention sont non seulement de puissants inhibiteurs de recapture de la sérotonine (activités équivalentes à celle de l'indalpine), mais en outre de puissants agents de libération de la sérotonine, encore plus actifs que la p-chloroamphétamine.

L'intérêt de ces composés réside dans le fait qu'ils induisent, comme la parachloroamphétamine, la libération de sérotonine sans pourtant présenter aucune des autres propriétés pharmacologiques caractéristiques des amphétaminiques (anorexie, hypermotilité).

*Propriétés toxicologiques*

La toxicité aiguë des produits a été déterminée chez la souris mâle $CO_1$ (Charles River) par voie orale. La $DL_{50}$ calculée, après 3 jours d'observation, par la méthode cumulative de J.J. Reed et coll. («Am. J. Hyg.», *27*, 493, 1938) est de 225 mg/kg pour le composé de l'exemple 1 et d'environ 200 mg/kg pour le composé de l'exemple 4.

Les composés selon l'invention sont atoxiques à 100 mg/kg et se comportent donc comme des substances relativement peu toxiques chez la souris.

*Application thérapeutique*

Les composés de l'invention et leurs sels pharmaceutiquement acceptables peuvent être utilisés en thérapeutique humaine sous forme de comprimés, capsules, gélules, suppositoires, solutions ingérables ou injectables, etc., comme régulateurs du tonus vasculaire sérotonino-dépendant, notamment pour le traitement des migraines, comme agents antithrombosants et comme médicaments thymoanaleptiques à action particulièrement rapide (à cause de leur action sur la libération de la sérotonine).

La posologie dépend des effets recherchés et de la voie d'administration utilisée. Par exemple, par voie orale, elle peut être comprise entre 15 et 250 mg de substance active par jour, avec des doses unitaires allant de 5 à 50 mg.

## Revendications

1. Les composés de formule générale:

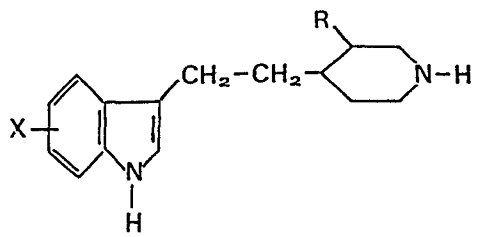

(II)

dans laquelle X représente un atome d'hydrogène ou d'halogène, et R représente un groupe alkyle ou alcényle possédant 1 à 3 atomes de carbone.

2. Composés selon la revedication 1, caractérisés en ce qu'ils possèdent la structure cis, leurs énantiomères et leurs racémiques.

3. Composés selon la revendication 1, caractérisés en ce qu'ils possèdent la structure trans, leurs énantiomères et leurs racémiques.

4. Procédé pour la préparation des composés selon la revendication 1, caractérisé en ce que l'on fait réagir un dérivé de l'indole de formule:

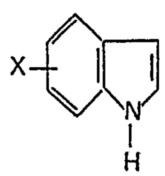

(III)

dans laquelle X représente un atome d'hydrogène ou d'halogène avec un dérivé métallique de formule R'Li dans laquelle R' représente un groupe alkyle, aryle ou dialkylamino, fait réagir le lithio-3 indole ainsi obtenu avec le dérivé de pipéridine de formule:

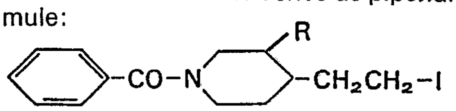

(V)

dans laquelle R représente un groupe alkyle ou alcényle possédant 1 à 3 atomes de carbone, et hydrolyse le [(benzoyl-1 alkyl- ou alcényl-3 pipéridyl-4)-2 éthyl]-3 indole (VI) ainsi obtenu.

5. Le composé de formule V pour la mise en œuvre du procédé revendiqué selon la revendication 4.

6. Procédé pour la préparation des composés revendiqués selon la revendication 1, caractérisé en ce que les dérivés cétoniques de formule générale:

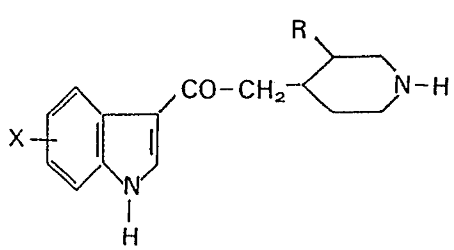

dans laquelle R et X ont les mêmes significations que dans la revendication 1 sont soumis à une réduction.

7. En tant qu'agents inhibiteurs de la recapture de la sérotonine et agents libérateurs de la sérotonine, utilisables comme médicaments spécialement comme thymoanaleptiques antimigraineux et antithrombosants, les composés selon la revendication 1 et leurs sels avec les acides pharmaceutiquement acceptables.

## Patentansprüche

1. Die Verbindungen der allgemeinen Formel:

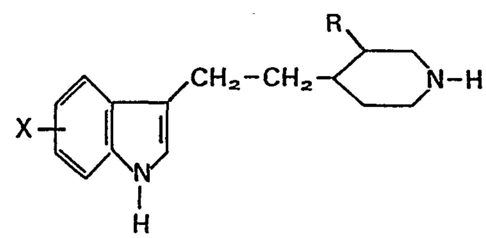

(II)

worin X ein Wasserstoff- oder Halogenatom bedeutet und R eine Alkyl- oder Alkenylgruppe mit 1 bis 3 Kohlenstoffatomen bedeutet.

2. Verbindungen gemäss Anspruch 1, dadurch gekennzeichnet, dass sie die cis-Struktur besitzen, ihre Enantiomeren und ihre Racemate.

3. Verbindungen gemäss Anspruch 1, dadurch gekennzeichnet, dass sie die trans-Struktur besitzen, ihre Enantiomeren und ihre Racemate.

4. Verfahren zur Herstellung der Verbindungen gemäss Anspruch 1, dadurch gekennzeichnet, dass man ein Derivat des Indols der Formel:

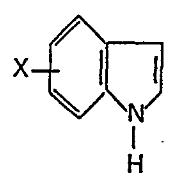

(III)

worin X ein Wasserstoff- oder Halogenatom bedeutet, mit einem Metallderivat der Formel R'Li, worin R' eine Alkyl-, Aryl- oder Dialkylaminogruppe bedeutet, umsetzt und dass man das so erhaltene 3-Lithioindol mit dem Piperidinderivat der Formel:

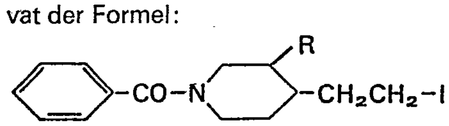

(V)

worin R eine Alkyl- oder Alkenylgruppe mit 1 bis 3 Kohlenstoffatomen bedeutet, umsetzt und das so erhaltene 3-[2-(1-Benzoyl-3-alkyl- oder -3-alkenyl-4-piperidyl)äthyl]indol (VI) hydrolysiert.

5. Die Verbindung der Formel (V) zum Einsatz bei dem gemäss Anspruch 4 beanspruchten Verfahren.

6. Verfahren zur Herstellung der Verbindungen gemäss Anspruch 1, dadurch gekennzeichnet, dass die Ketonderivate der allgemeinen Formel:

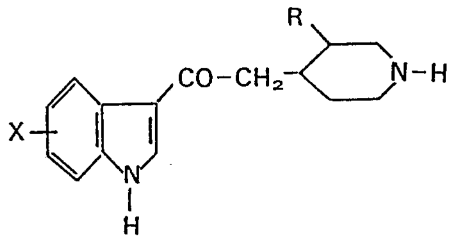

worin R und X dieselben Bedeutungen wie in Anspruch 1 haben, einer Reduktion unterworfen werden.

7. Als Inhibitoren der Wiederbindung des Serotonins und Mittel zur Freisetzung des Serotonins, die als Arzneimittel, besonders als Thymoanaleptika mit Antimigräne- und Antithrombosewirkung, anwendbar sind, die gemäss Anspruch 1 beanspruchten Verbindungen und ihre Salze mit pharmazeutisch annehmbaren Säuren.

## Claims

1. The compound of the general formula:

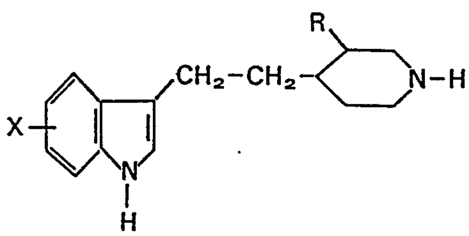

(II)

in which X represents a hydrogen or halogen atom and R represents an alkyl or alkenyl group possessing 1 to 3 carbon atoms.

2. Compounds according to Claim 1, characterised in that they have the cis-structure, as well as their enantiomers and their racemates.

3. Compounds according to Claim 1, characterised in that they have the trans-structure, as well as their enantiomers and their racemates.

4. Process for the preparation of the compounds according to Claim 1, characterised in that an indole derivative of the formula:

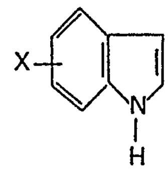

(III)

in which X represents a hydrogen atom or halogen atom is reacted with a metal derivative of the formula R'Li in which R' represents an alkyl, aryl or dialkylamino group, the 3-lithio-indole thus obtained is reacted with the piperidine derivative of the formula:

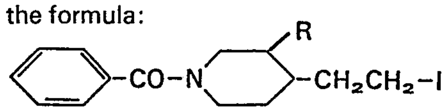

(V)

in which R represents an alkyl or alkenyl group possessing 1 to 3 carbon atoms and the 3-[2-(1-benzoyl-3-alkyl- or -3-alkenyl-4-piperidyl)ethyl] indole (VI) thus obtained is hydrolysed.

5. The compound of the Formula (V) for carrying out the process according to Claim 4.

6. Process for the preparation of the compounds according to Claim 1, characterised in that the ketone derivatives of the general formula:

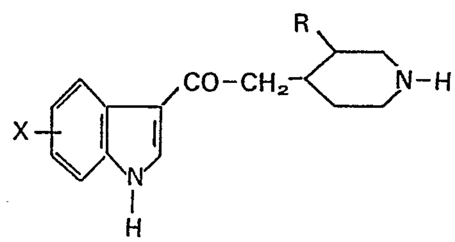

(X)

in which R and X have the same meanings as in Claim 1 are subjected to reduction.

7. As inhibitors of the recapture of serotonine and liberators of serotonine, which may be used as medicaments, especially as thymo-analeptic, antimigraine and anti-thrombosis agents, the compounds according to Claim 1 and their salts with pharmaceutically acceptable acids.